# EUROPÄISCHE PATENTSCHRIFT

(11) **EP 0 589 921 B1**
(45) Veröffentlichungstag und Bekanntmachung des Hinweises auf die Patenterteilung: **22.01.1997**
(21) Anmeldenummer: 92911099.7
(22) Anmeldetag: 02.06.1992
(51) Int. Cl.: A61K 35/78

(54) **PFLANZLICHE DICK- UND/ODER TROCKENEXTRAKTE**
CONCENTRATED AND/OR DRY PLANT EXTRACTS
EXTRAITS DE PLANTES SECS ET/OU CONCENTRES

(30) Priorität: 07.06.1991 DE 4118710; 19.06.1991 DE 4120370
(43) Veröffentlichungstag der Anmeldung: 06.04.1994
(73) Patentinhaber: Krewel Meuselbach GmbH, 53783 Eitorf (DE)
(72) Erfinder: SCHIERSTEDT, Detlef, D-5205 St. Augustin 3 (DE)
(74) Vertreter: Jönsson, Hans-Peter, Dr.Dipl.-Chem.
(86) Internationale Anmeldenummer: EP9201212
(87) Internationale Veröffentlichungsnummer: WO9221357

(56) Entgegenhaltungen:
- EP-A- 0 248 215
- EP-A- 0 370 284
- CHEMICAL ABSTRACTS, vol. 104, no. 13, 1986, Columbus, Ohio, US; abstract no. 106289G; GOERLER, K. ET AL.: 'IRIDOID DERIVATIVES FROM VITEX AGNUS-CASTUS & PLANTA MED. 1985, (6), 530-1', Seite 423

## Beschreibung

Gegenstand der Erfindung sind pflanzliche Dick- und/oder Trockenextrakte mit gleichem oder annähernd gleichem Wirkstoffspektrum eines entsprechenden Flüssigextraktes, deren Verwendung sowie ein Verfahren zu ihrer Herstellung.

Für die pharmazeutische Verarbeitung von pflanzlichen Drogen werden hauptsächlich Flüssigextrakte (Fluid-Extrakte und/oder Tinkturen), Dickextrakte (Spissum-Extrakte) und Trockenextrakte (Siccum-Extrakte) verwendet.

Durch die meisten Arzneibücher ist vorgegeben, für die Herstellung von Flüssigextrakten aus pflanzlichen Drogen in der Regel als Auszugsmittel ein Alkohol-Wasser-Gemisch einzusetzen.

Derartige wäßrig-ethanolische Auszüge entsprechen jedoch nicht den WHO-Empfehlungen und sind insbesondere für die Anwendung bei Alkoholikern und Kindern ungeeignet.

Oft enthalten pflanzliche Drogen eine Fülle medizinisch wirksamer, jedoch flüchtiger Bestandteile, so daß eine Entfernung des Auszugsmittels durch Destillation nicht ohne weiteres möglich ist. Halbfeste Dick- und/oder Trockenextrakte aus einem aus der gleichen Droge hergestellten Flüssigextrakt weisen daher erhebliche Unterschiede in der phytochemischen Zusammensetzung untereinander auf. Darüber hinaus ist in der Regel auch das Wirkstoffspektrum gegenüber dem Flüssigextrakt verändert. Nach den herkömmlich angewandten Verfahren geht ein Großteil der flüchtigen Drogenbestandteile bei der Herstellung von Dick- und/oder Trockenextrakten verloren. Klinisch-pharmakologische Aussagen, die mit ethanolhaltigen Flüssigextrakten gewonnen wurden, sind somit nicht ohne weiteres auf Präparate von Dick- und/oder Trockenextrakten zu übertragen.

Die Aufgabe der vorliegenden Erfindung besteht darin, pflanzliche Dick- und/oder Trockenextrakte mit gleichem oder annähernd gleichem Wirkstoffspektrum eines entsprechenden Flüssigextraktes zur Verfügung zu stellen.

Die vorgenannte Aufgabe wird gelöst mit pflanzlichen Dick- und/oder Trockenextrakten mit gleichem oder annähernd gleichem Wirkstoffspektrum eines entsprechenden Flüssigextraktes, die erhältlich sind durch destillative Entfernung des Auszugsmittels, Extraktion der flüchtigen Bestandteile mit einem toxikologisch unbedenklichen Extraktionsmittel und Vereinigung des Destillationsrückstandes mit der Extraktionsphase.

Mit Hilfe der vorliegenden Erfindung ist es möglich, feste Darreichungsformen von Flüssigextrakten zur Verfügung zu stellen, die ein gleiches oder annähernd gleiches Wirkstoffspektrum eines entsprechenden Flüssigextraktes aufweisen.

Die erfindungsgemäßen festen Darreichungsformen haben insbesondere bei Drogen mit schlechtem Geschmack einen erheblichen Patienten-Compliance-Vorteil gegenüber flüssigen Zubereitungen, da häufig aufgrund schlechter Compliance die Therapie vorzeitig vom Patienten abgebrochen wird. Darüber hinaus sind die erfindungsgemäßen Darreichungsformen im Gegensatz zu den Flüssig-Extrakt-Zubereitungen ethanolfrei, was inbesondere vorteilhaft ist für die Anwendung bei Alkoholikern und Kindern. Somit kann den WHO-Empfehlungen voll entsprochen werden. Auch erlaubt ein solcher Dick- und/oder Trockenextrakt die Herstellung von ethanolfreien Flüssigextrakten.

In einer bevorzugten Ausführungsform der vorliegenden Erfindung geht man von Flüssigextrakten aus, die als Auszugsmittel Wasser und/oder einen kurzkettigen aliphatischen Alkohol mit 1 bis 4 C-Atomen, insbesondere Ethanol, enthalten. Die Verwendung von Alkohol-Wasser-Gemischen in verschiedensten Mengenbereichen ist im Stand der Technik besonders verbreitet.

Bei der Wahl des Extraktionsmittels bestehen prinzipiell keine besonderen Einschränkungen, jedoch ist ein toxikologisch unbedenkliches Extraktionsmittel erforderlich, da dieses Extraktionsmittel Bestandteil des Dick- und/oder Trockenextraktes bleibt. Übliche Extraktionsmittel sollten mit dem gegebenenfalls mit Wasser verdünnten Flüssigextrakt nicht mischbar sein, um eine schnelle und einfache Extraktion zu erlauben. Bevorzugte Extraktionsmittel im Sinne der vorliegenden Erfindung sind ausgewählt aus organischen Carbonsäureestern mit
a) 6 bis 22 C-Atomen im gesättigten, einfach und/oder mehrfach ungesattigten, geradkettigen oder verzweigtkettigen Carbonsäurerest mit wenigstens einer oder mehreren Carbonsäurefunktionen und
b) Mono-, Di-, Oligo- und Polyalkoholen mit wenigstens 2 C-Atomen im Alkoholrest.

Besonders bevorzugt im Sinne der vorliegenden Erfindung sind die Ester nativer Fettsäuren. In diesem Sinne besonders bevorzugt ist der Carbonsäurerest der Carbonsäureester ausgewählt aus Caprylsäure, Caprinsäure, Laurylsäure, Myristinsäure, Stearinsäure, Behensäure, Caproleinsäure, Lauroleinsäure, Myristoleinsäure, Ölsäure, Gadoleinsäure, Erucasäure, Linoleinsäure, Arachidonsäure, Clupanodonsäure, Adipinsäure, Azelainsäure, Sebacinsäure, Citronensäure und Aconitsäure.

In analoger Weise sind die Alkoholreste der Carbonsäureester bevorzugterweise ausgewählt aus Ethanol, 1-Propanol, 2-Propanol, Ethylenglykol, Diethylenglykol, Triethylenglykol, Glycerin, Pentaerythrit, Pentiten und Hexiten.

Aufgrund der besonderen Lösungsmitteleigenschaften sind die Extraktionsmittel im Sinne der vorliegenden Erfindung besonders dann geeignet, wenn diese ausgewählt sind aus Fettsäureestern des 2-Propanols, insbesondere Isopropylmyristat, und Fettsäureestern des Glycerins.

Die erfindungsgemäßen Extrakte können weiterhin pharmazeutische Hilfsstoffe, Trägerstoffe und/oder Sprengmittel enthalten. Hierbei ist insbesondere bevorzugt, daß die Dick- und/oder Trockenextrakte hochdisperses Siliciumdioxid, Stärke, Stärkederivate, Cellulose, Cellulosederivate, Poly(1-vinyl-2-pyrrolidon), Poly(1-vinyl-2-pyrrolidon)-Derivate, Einfach- und/oder Mehrfachzucker, insbesondere Saccharose, Lactose, Zuckeralkohole, insbesondere Mannit, Xylit, Sorbit, Polyethylenglykol, Cyclodextrine, Propylenglykol, Carbonsäureester und/oder physiologisch verträgliche Tenside, insbesondere Polysorbate, enthalten.

Diese Hilfsstoffe dienen der weiteren Verarbeitung und stellen pharmazeutisch übliche Hilfsstoffe dar. Die Hilfsstoffe können einzeln oder in Mischung eingesetzt werden.

Besonders bevorzugte Extrakte im Sinne der vorliegenden Erfindung sind gekennzeichnet durch einen Flüssigextrakt aus wäßrig-ethanolischem Vitex Agnus Castus.

Ein weiterer Gegenstand der vorliegenden Erfindung besteht in der Verwendung der Dick- und/oder Trockenextrakte zur Herstellung von alkoholfreien Flüssigextrakten, Tabletten, Kapseln und/oder Granulaten. Die Herstellung derartiger fester Darreichungsformen von Wirkstoffen ist prinzipiell dem Fachmann auf dem hier vorliegenden Gebiet bekannt und bedarf keiner näheren Erläuterung.

Besonders bevorzugt im Sinne der vorliegenden Erfindung ist jedoch die Herstellung von alkoholfreien Flüssigextrakten, insbesondere auf der Basis von Glycofurol und/oder Propylenglykol.

In analoger Weise ist es mit Hilfe der vorliegenden Erfindung möglich, zur Herstellung von Kapseln Weich- oder Hartgelatine einzusetzen.

Ein weiterer Gegenstand der vorliegenden Erfindung betrifft ein Verfahren zur Herstellung der Dick- und/oder Trockenextrakte. In einer ersten Ausführungsform geht man davon aus, daß man zunächst das Auszugsmittel des Flüssigextraktes destillativ entfernt, dann das gegebenenfalls mit Wasser verdünnte Destillat der Extraktion mit einem toxikologisch unbedenklichen Extraktionsmittel unterwirft und anschließend den Destillationsrückstand mit der Extraktionsphase vereinigt.

Überraschenderweise wurde gefunden, daß beispielsweise durch Abdestillieren des Ethanol-Wasser-Gemisches aus Flüssigextrakten und anschließende Extraktion des gegebenenfalls mit Wasser verdünnten Destillats mit einem geeigneten Extraktionsmittel die flüchtigen Drogenbestandteile aus der Alkohol-Wasser-Phase praktisch vollständig extrahiert werden konnten.

Nach dem Abtrennen der Extraktionsphase und anschließender Vereinigung derselben mit dem Destillationsrückstand erhält man gegebenenfalls durch Zugabe von geeigneten Hilfsstoffen einen Dick- und/oder Trockenextrakt, mit gleichem oder annähernd gleichem Wirkstoffspektrum, wie im ursprünglichen Flüssigextrakt. Bedingt durch die Eigenschaften des Extraktionsmittels und Zugabe von weiteren Hilfsstoffen, beispielsweise hochdispersem Siliciumdioxid, kann verhindert werden, daß die flüchtigen Bestandteile bei der späteren Weiterverarbeitung zu Pharmazeutika in relevantem Ausmaß entweichen.

In gleicher Weise wurde gefunden, daß ein Verfahren zur Herstellung der Dick- und/oder Trockenextrakte mit gleichem Ergebnis zur Verfügung gestellt werden kann, wenn man zunächst den gegebenenfalls mit Wasser verdünnten Flüssigextrakt der Extraktion mit einem toxikologisch unbedenklichen Extraktionsmittel unterwirft, dann das Auszugsmittel destillativ entfernt und anschließend den Destillationsrückstand und die Extraktionsphase vereinigt.

Es wurde gefunden, daß durch Extraktion eines beispielsweise wäßrig-ethanolischen, gegebenenfalls mit Wasser verdünnten Flüssigextraktes mit einem geeigneten, nicht mischbaren Extraktionsmittel die flüchtigen Drogenbestandteile praktisch vollkommen extrahiert werden können.

Nach dem Abtrennen der Extraktionsphase und dem Eindampfen der wäßrig-ethanolischen Flüssigextraktphase wird der resultierende Eindampfrückstand mit der Extraktionsphase, gegebenenfalls unter Zusatz von geeigneten Hilfsstoffen, zu einem Dick- und/oder Trockenextrakt weiterverarbeitet. Das Wirkstoffspektrum ist praktisch mit dem des Ausgangs-Flüssigextraktes vergleichbar. Bei thermolablien Extrakten kann das erfindungsgemäße Verfahren auch unter verminderter Temperatur und vermindertem Druck durchgeführt werden.

### Beispiele

### Beispiel 1

### Herstellung eines Trockenextraktes aus wäßrig-ethanolischem Vitex Agnus Castus-Flüssigextrakt

200 g alkoholisch-wäßriger Flüssigextrakt (Tinktur) 1 zu 5 aus Mönchspfefferfrüchten mit einem Alkoholgehalt (Ethanol) von 58 Vol. % wurden in einen Rotationsverdampfer gegeben und bei Atmosphärendruck destilliert.

Anschließend wurde das Destillat mit 20 g Isopropylmyristat und 120 ml gesättigter Kochsalzlösung versetzt und geschüttelt. Nach dem Absetzen der Isopropylmyristat-Phase wurde diese abgetrennt und mit dem Destillationsrückstand vereinigt. Den vereinigten Phasen wurden 130 g Lactose, 23 g hochdisperses Siliciumdioxid, 10 g Propylenglykol und 2 g Polysorbat 20 zugegeben.

Nach intensivem Mischen entstand ein Trockenextrakt.

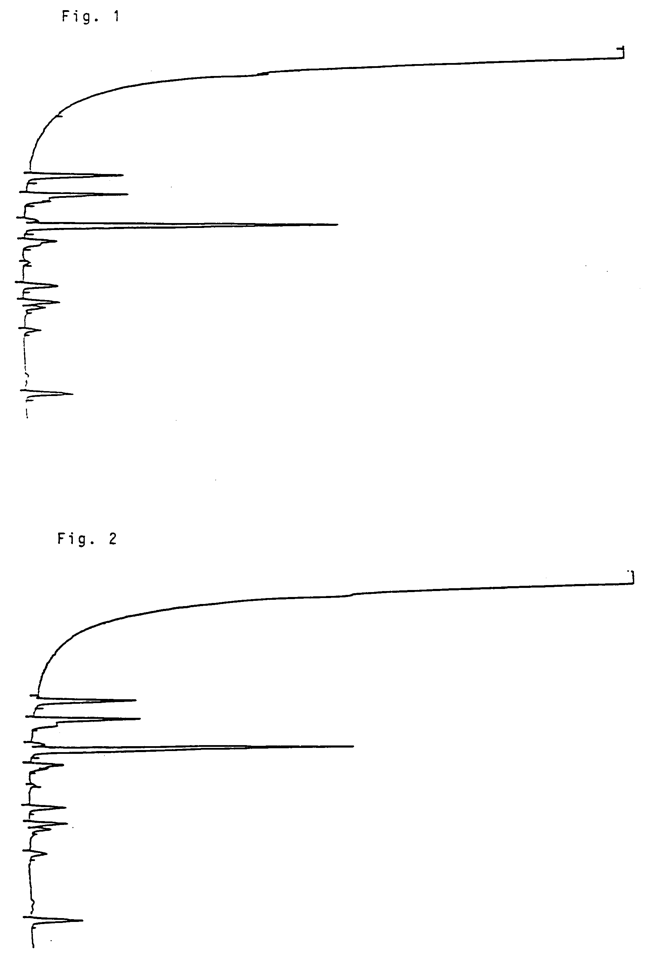

Der Fig. 1 ist ein Gaschromatogramm des so hergestellten Trockenextraktes, ausgehend von ethanolischwäßrigem Flüssigextrakt aus Vitex Agnus Castus, zu entnehmen.

Die Fig. 2 gibt ein Gaschromatogramm von ethanolischwäßrigem Flüssigextrakt von Vitex Agnus Castus wieder.

### Beispiel 2

### Herstellung von alkoholfreiem Flüssigextrakt

200 g des oben genannten alkoholisch-wäßrigen Flüssigextraktes aus Vitex Agnus Castus wurden in einem Rotationsverdampfer wie oben destilliert. Dann wurde auch hier das Destillat mit 20 g Isopropylmyristat und 120 ml gesättigter Kochsalzlösung versetzt und geschüttelt. Nach dem Absetzen der Isopropylmyristat-Phase wurde diese abgetrennt und mit dem Destillationsrückstand vereinigt. Anschließend wurde mit Glycofurol auf das ursprüngliche Volumen von 200 ml wiederaufgefüllt.

### Beispiel 3

### Herstellung von Trockenextrakt

200 g des oben genannten ethanolisch-wäßrigen Flüssigextraktes aus Vitex Agnus Castus wurden mit 20 g Isopropylmyristat, 100 ml Wasser und 4 g Natriumchlorid versetzt. Nach dem Schütteln und Absetzen der Isopropylmyristat-Phase wurde die ethanolisch-wäßrige Phase abgetrennt und eingedampft. Der Eindampfrückstand wurde mit der Isopropylmyristat-Phase vereinigt und mit 130 g Lactose, 23 g hochdispersem Siliciumdioxid, 10 g Propylenglykol und 2 g Polysorbat 20 versetzt. Nach intensivem Mischen entstand ein Trockenextrakt.

### Beispiel 4

### Herstellung einer pharmazeutischen Zubereitung

200 g des oben genannten ethanolisch-wäßrigen Flüssigextraktes aus Vitex Agnus Castus wurden mit 20 g Isopropylmyristat, 100 ml Wasser und 0,5 g Natriumchlorid versetzt. Nach dem Schütteln und Absetzen der Isopropylmyristat-Phase wurde die ethanolisch-wäßrige Phase abgetrennt und eingedampft. Der Eindampfrückstand wurde mit der Isopropylmyristat-Phase vereinigt und mit Glycofurol auf das ursprüngliche Volumen von 200 ml wiederaufgefüllt und filtriert. Anschließend wurde der Extrakt mit 12 g Aromatisierungsmittel versetzt und mit Propylenglykol auf 2000 ml verdünnt.

## Patentansprüche

1. Pflanzliche Dick- und/oder Trockenextrakte von pflanzlichen Drogen mit gleichem oder annähernd gleichem Wirkstoffspektrum eines entsprechenden Flüssigextraktes, erhältlich durch destillative Entfernung des Auszugsmittels des Flüssigextrakts, Extraktion der flüchtigen Bestandteile aus dem Destillat mit einem toxikologisch unbedenklichen Extraktionsmittel und Vereinigung des Destillationsrückstandes mit der Extraktionsphase des Destillats.

2. Extrakte nach Anspruch 1, dadurch gekennzeichnet, daß das Auszugsmittel der pflanzlichen Drogen Wasser und/oder einen kurzkettigen aliphatischen Alkohol mit 1 bis 4 C-Atomen, insbesondere Ethanol, enthält.

3. Extrakte nach Anspruch 1 oder 2, dadurch gekennzeichnet, daß das Extraktionsmittel ausgewählt ist aus organischen Carbonsäureestern mit
a) 6 bis 22 C-Atomen im gesättigten, einfach und/oder mehrfach ungesättigten, geradkettigen oder verzweigtkettigen Carbonsäurerest mit wenigstens einer oder mehreren Carbonsäurefunktionen und
b) Mono-, Di-, Oligo- und Polyalkoholen mit wenigstens 2 C-Atomen im Alkoholrest.

4. Extrakte nach einem oder mehreren der Ansprüche 1 bis 3, dadurch gekennzeichnet, daß der Carbonsäurerest der Carbonsäureester ausgewählt ist aus Caprylsäure, Caprinsäure, Laurylsäure, Myristinsäure, Stearinsäure, Behensäuren, Caproleinsäure, Lauroleinsäure, Myristoleinsäure, Ölsäure, Gadoleinsäure, Erucasäure, Linoleinsäure, Arachidonsäure, Clupanodonsäure, Adipinsäure, Azelainsäure, Sebacinsäure, Citronensäure und Aconitsäure.

5. Extrakte nach einem oder mehreren der Ansprüche 1 bis 4, dadurch gekennzeichnet, daß die Alkoholreste der Carbonsäureester ausgewählt sind aus Ethanol, 1-Propanol, 2-Propanol, Ethylenglykol, Diethylenglykol, Triethylenglykol, Glycerin, Pentaerythrit, Pentiten und Hexiten.

6. Extrakte nach einem oder mehreren der Ansprüche 1 bis 5, dadurch gekennzeichnet, daß die Extraktionsmittel ausgewählt sind aus Fettsäureestern des 2-Propanols, insbesondere Isopropylmyristat, und Fettsäureestern des Glycerins.

7. Extrakte nach einem oder mehreren der Ansprüche 1 bis 6, dadurch gekennzeichnet, daß sie weiterhin pharmazeutische Hilfsstoffe, Trägerstoffe und/oder Sprengmittel enthalten.

8. Extrakte nach Anspruch 7, dadurch gekennzeichnet, daß sie hochdisperses Siliciumdioxid, Stärke, Stärkederivate, Cellulose, Cellulosederivate, Poly(1-vinyl-2-pyrrolidon), Poly(1-vinyl-2-pyrrolidon)-Derivate, Einfach- und/oder Mehrfachzucker, insbesondere Saccharose, Lactose, Zuckeralkohole, insbesondere Mannit, Xylit, Sorbit, Polyethylenglykol, Cyclodextrine, Propylenglykol, Carbonsäureester, physiologisch verträgliche Tenside, insbesondere Polysorbate, enthalten.

9. Extrakte nach einem oder mehreren der Ansprüche 1 bis 8, gekennzeichnet durch einen Flüssigextrakt aus wäßrig-ethanolischem Vitex Agnus Castus.

10. Verwendung der Dick- und/oder Trockenextrakte nach einem oder mehreren der Ansprüche 1 bis 9 zur Herstellung von alkoholfreien Flüssigextrakten, Tabletten, Kapseln und/oder Granulaten.

11. Verwendung nach Anspruch 10, dadurch gekennzeichnet, daß man zur Herstellung von alkoholfreien Flüssigextrakten Glycofurol und/oder Propylenglykol einsetzt.

12. Verwendung nach Anspruch 10, dadurch gekennzeichnet, daß man zur Herstellung von Kapseln Weich- oder Hartgelatine einsetzt.

13. Verfahren zur Herstellung von Dick- und/oder Trockenextrakte von pflanzlichen Drogen nach einem oder mehreren der Ansprüche 1 bis 9, dadurch gekennzeichnet, daß man zunächst das Auszugsmittel des Flüssigextraktes destillativ entfernt, dann das Destillat enthaltend flüchtige Bestandteile der Extraktion mit einem toxikologischen unbedenklichen Extraktionsmittel unterwirft und anschließend den Destillationsrückstand mit der Extraktionsphase vereinigt.

14. Verfahren zur Herstellung von Dick- und/oder Trockenextrakte von pflanzlichen Drogen nach einem oder mehreren der Ansprüche 1 bis 9, dadurch gekennzeichnet, daß man zunächst den Flüssigextrakt der Extraktion mit einem toxikologisch unbedenklichen Extraktionsmittel unterwirft, dann das Auszugsmittel destillativ entfernt und anschließend den Destillationsrückstand mit der Extraktionsphase vereinigt.

15. Verfahren nach Anspruch 13 oder 14, dadurch gekennzeichnet, daß das Auszugsmittel unter vermindertem Atmosphärendruck destillativ entfernt wird.

16. Verfahren nach einem oder mehreren der Ansprüche 13 bis 15, dadurch gekennzeichnet, daß man Extraktionshilfsmittel zusetzt.

17. Verfahren nach Anspruch 16, dadurch gekennzeichnet, daß man den Flüssigextrakt mit Wasser verdünnt.

## Claims

1. Concentrated and/or dry plant extracts of vegetable drugs having the same or about the same spectrum of active ingredients as a corresponding fluid extract, obtainable by removing the extractant from said fluid extract by distillation, extracting the volatile components from the distillate with a toxicologically acceptable extracting agent, and combining the distillation residue with the extraction phase of the destillate.

2. Extracts according to claim 1, characterized in that said extractant of the vegetable drugs contains water and/or a short-chain aliphatic alcohol having from 1 to 4 carbon atoms, especially ethanol.

3. Extracts according to claim 1 or 2, characterized in that said extracting agent is selected from organic carboxylic acid esters comprising
a) from 6 to 22 carbon atoms in the saturated, mono- and/or poly-unsaturated, straight or branched chain carboxylic acid residue having at least one or more carboxylic acid functionalities; and
b) mono-, di-, oligo- and poly-alcohols having at least 2 carbon atoms in the alcohol residue.

4. Extracts according to one or more of claims 1 to 3, characterized in that said carboxylic acid residue of said carboxylic acid esters is selected from caprylic acid, capric acid, lauric acid, myristic acid, stearic acid, behenic acids, caproleic acid, lauroleic acid, myristoleic acid, oleic acid, gadoleic acid, erucic acid, linoleic acid, arachidonic acid, clupanodonic acid, adipic acid, azelaic acid, sebacic acid, citric acid and aconitic acid.

5. Extracts according to one or more of claims 1 to 4, characterized in that said alcohol residues of said carboxylic acid esters are selected from ethanol, 1-propanol, 2-propanol, ethylene glycol, diethylene glycol, triethylene glycol, glycerol, pentaerythritol, pentitols and hexitols.

6. Extracts according to one or more of claims 1 to 5, characterized in that said extracting agents are selected from fatty acid esters of 2-propanol, in particular isopropyl myristate, and fatty acid esters of glycerol.

7. Extracts according to one or more of claims 1 to 6, characterized by further containing pharmaceutic excipients, vehicles and/or disintegrants.

8. Extracts according to claim 7, characterized by containing highly dispersed silica, starch, starch derivatives, cellulose, cellulose derivatives, poly(1-vinyl-2-pyrrolidone), poly(1-vinyl-2-pyrrolidone) derivatives, mono- and/or polysaccharides, in particular saccharose, lactose, sugar alcohols, in particular mannitol, xylitol, sorbitol, polyethylene glycol, cyclodextrines, propylene glycol, carboxylic acid esters, physiologically acceptable surfactants, in particular polysorbates.

9. Extracts according to one or more of claims 1 to 8, characterized in that said fluid extract is a water/ethanol fluid extract from Vitex Agnus Castus.

10. Use of the concentrated and/or dry extracts according to one or more of claims 1 to 9 for the preparation of alcohol-free fluid extracts, tablets, capsules and/or granulates.

11. The use according to claim 10, characterized in that glycofurol and/or propylene glycol is employed for the preparation of alcohol-free fluid extracts.

12. The use according to claim 10, characterized in that soft or hard gelatin is employed for the preparation of capsules.

13. A process for the preparation of concentrated and/or dry extracts of vegetable drugs according to one or more of claims 1 to 9, characterized in that the extractant is first removed from said fluid extract by distillation, the distillate containing volatile components is then subjected to extraction with a toxicologically acceptable extracting agent, and the distillation residue is subsequently combined with the extraction phase.

14. A process for the preparation of concentrated and/or dry extracts of vegetable drugs according to one or more of claims 1 to 9, characterized in that said fluid extract is first subjected to extraction with a toxicologically acceptable extracting agent, the extractant is then removed by distillation, and the distillation residue is subsequently combined with the extraction phase.

15. A process according to claim 13 or 14, characterized in that said extractant is removed by distillation under reduced atmospheric pressure.

16. A process according to one or more of claims 13 to 15, characterized in that extraction aids are added.

17. A process according to claim 16, characterized in that said fluid extract is diluted with water.

## Revendications

1. Extraits de plantes concentrés et/ou secs de drogues végétales avec le même ou presque le même spectre d'activité qu'un extrait liquide correspondant, extraits susceptibles d'être obtenus en séparant par distillation le milieu d'extraction de l'extrait liquide, en extrayant du distillat les constituants volatils au moyen d'un agent d'extraction toxicologiquement sans danger et en combinant le résidu de distillation avec la phase d'extraction du distillat.

2. Extraits selon la revendication 1, caractérisés en ce que le milieu d'extraction des drogues végétales contient de l'eau et/ou un alcool aliphatique à chaîne courte ayant 1 à 4 atomes de carbone, en particulier l'éthanol.

3. Extraits selon la revendication 1 ou 2, caractérisés en ce que l'agent d'extraction est sélectionné parmi les esters d'acides carboxyliques organiques avec :
a) 6 à 22 atomes de carbone dans le radical acide carboxylique saturé, une ou plusieurs fois insaturé, à chaîne droite ou ramifiée, comportant au moins une ou plusieurs fonctions acide carboxylique, et
b) des mono-, dl, oligo- et polyalcools ayant au moins 2 atomes de carbone dans le radical alcool.

4. Extraits selon une ou plusieurs des revendications 1 à 3, caractérisés en ce que le radical acide carboxylique de l'ester d'acide carboxylique est sélectionné parmi l'acide caprylique, l'acide caprique, l'acide laurique, l'acide myristique, l'acide stéarique, l'acide béhénique, l'acide caproléinique, l'acide lauroléinique, l'acide myristoléinique, l'acide oléique, l'acide gadoléinique, l'acide érucique, l'acide linoléinique, l'acide arachidonique, l'acide clupadoïque, l'acide adipique, l'acide azélaïque, l'acide sébacique, l'acide citrique et l'acide aconitique.

5. Extraits selon une ou plusieurs des revendications 1 à 4, caractérisés en ce que les radicaux alcool de l'ester d'acide carboxylique sont sélectionnés parmi l'éthanol, le 1-propanol, le 2-propanol, l'éthylène glycol, le diéthylène glycol, le triéthylène glycol, le glycérol, le pentaérythritol, les pentites et les hexites.

6. Extraits selon une ou plusieurs des revendications 1 à 5, caractérisés en ce que les agents d'extraction sont sélectionnés parmi les esters d'acides gras avec le 2-propanol, en particulier le myristate d'isopropyle, et les esters d'acides gras avec le glycérol.

7. Extraits selon une ou plusieurs des revendications 1 à 6, caractérisés en ce qu'ils contiennent par ailleurs des agents auxiliaires pharmaceutiques, des substrats et/ou des agents de dispersion.

8. Extraits selon la revendication 7, caractérisés en ce qu'ils contiennent, du dioxyde de silicium à l'état fortement dispersé, de l'amidon, des dérivés d'amidon, de la cellulose, des dérivés cellulosiques, de la poly(1-vinyl-2-pyrrolidone), des dérivés de poly(1-vinyl-2-pyrrolidone), des sucres simples et/ou multiples, en particulier le saccharose, le lactose, des alcools de sucres, en particulier le mannitol, le xylitol, le sorbitol, le polyéthylène glycol, la cyclodextrine, le propylène glycol, des esters d'acides carboxyliques, des agents tensioactifs physiologiquement compatibles, en particulier des polysorbates.

9. Extraits selon une ou plusieurs des revendications 1 à 8, caractérisés par un extrait liquide tiré du Vitex Agnus Castus en solution dans de l'eau et de l'éthanol.

10. Utilisation d'extraits de plantes concentrés et/ou secs selon une ou plusieurs des revendications 1 à 9 pour la fabrication d'extraits liquides sans alcool, de comprimés, de capsules et/ou de granulés.

11. Utilisation selon la revendication 10, caractérisée en ce qu'on utilise du glycofurol et/ou du propylène glycol pour la fabrication d'extraits liquides sans alcool.

12. Utilisation selon la revendication 10, caractérisée en ce qu'on utilise de la gélatine dure ou molle pour la fabrication de capsules.

13. Procédé de fabrication d'extraits de plantes concentrés et/ou secs de drogues végétales selon une ou plusieurs des revendications 1 à 9, caractérisé en ce qu'on sépare tout d'abord le milieu d'extraction de l'extrait liquide par distillation, on soumet ensuite le distillat contenant des constituants volatils à une extraction par un agent d'extraction toxicologiquement sans danger et on combine ensuite le résidu de distillation avec la phase d'extraction.

14. Procédé de fabrication d'extraits de plantes concentrés et/ou secs de drogues végétales selon une ou plusieurs des revendications 1 à 9, caractérisé en ce qu'on soumet tout d'abord l'extrait liquide à une extraction par un agent d'extraction toxicologiquement sans danger, on sépare ensuite le milieu d'extraction par distillation et on combine ensuite le résidu de distillation avec la phase d'extraction.

15. Procédé selon la revendication 13 ou 14, caractérisé en ce que le milieu d'extraction est séparé par distillation sous pression atmosphérique réduite.

16. Procédé selon une ou plusieurs des revendications 13 à 15, caractérisé en ce qu'on ajoute un agent d'extraction auxiliaire.

17. Procédé selon la revendication 16, caractérisé en ce qu'on dilue l'extrait liquide avec de l'eau.
